(19) Europäisches Patentamt

European Patent Office

Office européen des brevets

(11) **EP 1 046 410 A2**

(12) **EUROPÄISCHE PATENTANMELDUNG**

(43) Veröffentlichungstag:
   **25.10.2000  Patentblatt 2000/43**

(51) Int. Cl.[7]: **A61P 19/00**, A61K 33/06

(21) Anmeldenummer: **00250128.6**

(22) Anmeldetag: **20.04.2000**

(84) Benannte Vertragsstaaten:
   **AT BE CH CY DE DK ES FI FR GB GR IE IT LI LU MC NL PT SE**
   Benannte Erstreckungsstaaten:
   **AL LT LV MK RO SI**

(30) Priorität: **20.04.1999 DE 19917705**

(71) Anmelder: **Vitasyn GmbH**
   **10789 Berlin (DE)**

(72) Erfinder:
   • **Markwitan, Arno, Dr.**
     **10829 Berlin (DE)**
   • **Lindenbacher, Michael**
     **14089 Berlin (DE)**

(74) Vertreter:
   **Scholz, Hartmut, Dipl.-Ing.**
   **Patentanwalt**
   **Rheinstrasse 64**
   **12159 Berlin (DE)**

(54) **Calcium- und Magnesiumhaltige Phosphatbinder zur Therapie von Hyperphosphatämie**

(57)    Mittel zur Senkung der Hypercalcämie und der Hypermagnesämie bei der Anwendung calciuum- oder magnesiumhaltiger Phosphatbinder zur Therapie von Hyperphosphatämie. Durch eine gleichzeitige Applikation phosphatbindender, unter physiologischen Bedingungen löslichen Calcium- und Magnesiumverbindungen in einem geeigneten Mol-Verhältnis, bei dem eine wechselseitige Resorptionshemmung auftritt, wird deren Resorption im Intestinaltrakt behindert.

EP 1 046 410 A2

**Beschreibung**

Die Erfindung betrifft die Verwendung calcium- und magnesiumhaltiger Phosphatbinder zur Therapie von Hyperphosphatämie.

Bei Niereninsuffienz, insbesondere im terminalen, dialysepflichtigen Stadium kommt es im menschlichen Körper zu einer Störung der renalen Phosphataausscheidung. Durch diese Störung der renalen Phosphataausscheidung kann sich eine Hyperphosphatämie entwickeln, die unbehandelt wiederum zu schweren Knochenerkrankungen in Einheit mit einem sekundären Hyperparathyreoidismus führen kann. Zur Senkung der Blutphosphatwerte ist es beispielsweise aus der DE 39 36 119 C2 bekannt, Calcium- und Magnesiumverbindungen einzusetzen, die unter physiologischen Bedingungen im Gastrointestinaltrakt dissoziieren und zu einer gastrointestinalen Phosphatbindung führen. Dabei bilden zweiwertige Calcium- bzw. Magnesium-Ionen im Gastrointestinaltrakt mit der resorbierbaren Phosphatmenge nach den Gleichungen

$$3Ca^{2+} + 2(PO_4)^{3-} = Ca_3(PO_4)_2 \text{ bzw. } 3Mg^{2+} + 2(PO_4)^{3-} = Mg_3(PO_4)_2$$

schwerlösliche Calcium- bzw. Magnesiumverbindungen, die mit den Faeces ausgeschieden werden können. Die resorbierbare Phosphatmenge wird dadurch reduziert und der erhöhte Blutsphosphatspiegel gesenkt.

Durch diese Maßnahmen wird zwar die resorbierbare Phosphatmenge gesenkt und der Blutphosphatspiegel sinkt, gleichzeitig können jedoch als Nebenwirkungen Hypercalcämien bzw. Hypermagnesämien auftreten, da die zur Phosphatbindung erforderlichen Mengen an elementarem Calcium und Magnesium weit über den ernährungsphysiologischen Dosen liegen.

Aufgabe der vorliegenden Erfindung ist es, ein Mittel zur Therapie der Hyperphosphatämie bei gleichzeitiger Vermeidung der Hypercalcämie und der Hypermagnesämie zu schaffen.

Gelöst wird diese Aufgabe durch die Maßnahmen gemäß Anspruch 1, insbesondere durch eine gleichzeitige Applikation von phosphatbindenden und unter physiologischen Bedingungen löslichen Calcium- und Magnesiumverbindungen, wobei das Mol-Verhältnis Calcium zu Magnesium von 1:0,2 bis 1:20.

Durch diese Maßnahmen wird ein Mittel angewendet, bei dem sich die an sich bekannte Calciumresorption und die Magnesiumresorption durch die gleichzeitige Anwesenheit des jeweils anderen Ions gegenseitig behindern. Weitere vorteilhafte Maßnahmen sind in den Unteransprüchen beschrieben.

Die Erfindung besteht darin, daß der ernährungsphysiologische, an sich negative Effekt, nämlich die wechselseitige Hemmung der Calcium- bzw. Magnesiumresorption als gezielte Therapie ausgenutzt wird. Die Hemmung der Calcium- und Magnesiumresorption ist zeitlich begrenzt.

Durch die gleichzeitige Applikation von Calcium und Magnesium in einer erheblichen Überdosis tritt durch die wechselseitige Hemmung eine zeitliche Verzögerung der Resorptionen auf. Diese zeitliche Verzögerung der Resorptionen reicht aus, um die bei der Phosphatbindung überschüssigen Calcium- und Magnesium-Ionen weitgehend unresorbiert durch den Intestinaltrakt zu fördern, daß sie nahezu vollständig mit den Faeces ausgeschieden werden können.

Geeignet sind alle Calcium- und/oder Magnesiumverbindungen, die unter den im menschlichen Gastrointestinaltrakt anzutreffenden Bedingungen Calcium- und/oder Magnesium-Ionen freisetzen. Diese können als Mischungen von Calcium- und Magnesiumsalzen mit gleichen oder unterschiedlichen Anionen eingesetzt werden. Eine Anwendung von Calcium mal Magnesium-Doppelsalzen oder deren Mischungen ist ebenso möglich.

Insbesondere sind Calciumacetat, Calciumalginat, Calciumcarbonat, Calciumcitrat, Calciumfumarat, Calciumgluconat, Calciumglutamat, Calciumhydroxid, Calciumlactat, Calciummalate, Calciumphytat, Calciumsilicate, Calciumsuccinat, Calciumtartat, die Calciumsalze von Aminosäuren, die Calciumsalze der Ketoanaloga von Aminosäuren oder die Calciumsalze von organischen Säuren, die im pflanzlichen, tierischen und menschlichen Stoffwechsel angetroffen werden, zur Anwendung vorgesehen.

Ebenso sind Magnesiumacetat, Magnesiumalginat, Magnesiumcarbonat, Magnesiumcitrat, Magnesiumfumarat, Magnesiumgluconat, Magnesiumglutamat, Magnesiumhydroxid, Magnesiumlactat, Magnesiummalate, Magnesiumphytat, Magnesiumsilicate, Magnesiumsuccinat, Magnesiumtartat, die Magnesiumsalze von Aminosäuren, die Magnesiumsalze der Ketoanaloga von Aminosäuren oder die Magnesiumsalze von organischen Säuren, die im pflanzlichen, tierischen und menschlichen Stoffwechsel angetroffen werden, zur Anwendung geeignet.

Als besonders geeignet zur erfindungsgemäßen Erzielung der wechselseitigen Hemmung Calcium und Magnesium in einer Darreichungsform hat sich ein molares Verhältnis von Calcium zu Magnesium wie 1 zu 0,2 bis 1 zu 20 als besonders geeignet erwiesen. Dies bedeutet, daß 1 mmol Calcium mit 0,2 mmol bis 20 mmol Magnesium zusammen appliziert werden. In absoluten Maßeinheiten würde das 40,8 mg Calcium zusammen mit 4,86 mg bis 486 mg Magnesium bedeuten.

Der absolute Gehalt der Darreichungsformen an Calcium und Magnesium kann so groß sein, daß eine oder mehrere Einheiten täglich eingenommen werden müssen, um die angestrebte Senkung der Hyperphosphatämie zu erreichen.

Geeignet sind alle oralen Darreichungsfomen wie Tabletten, Filmtabletten, Dragees, Kapseln aus Hart- oder Weichgelatine oder sonstigen Umhüllungsstoffen in magensaftlöslicher oder magensaftresistenter Form. Ebenfalls sind Lutsch- oder Kautabletten geeignet. Weiterhin können magensaftlösliche und magensaftresistente Granulate, Lösungen, Tropfen, Suspensionen, Säfte oder Gele zur Anwendung gelangen. Diese Darreichungsformen können zusätzlich zu den therapeutisch wirksamen Calcium- und Magnesiumverbindungen weitere galanisch notwendige, pharmazeutischen Regeln entsprechende Hilfsstoffe enthalten.

**Patentansprüche**

1. Verwendung calcium- und magnesiumhaltiger Phosphatbinder zur Therapie von Hyperphosphatämie, gekennzeichnet durch eine gleichzeitige Applikation von phosphatbindenden und unter physiologischen Bedingungen löslichen Calcium- und Magnesiumverbindungen, wobei das Mol-Verhältnis Calcium zu Magnesium von 1:0,2 bis 1:20.

2. Verwendung nach Anspruch 1, dadurch gekennzeichnet, daß sie in typischen oralen Darreichungsformen und inklusive zusätzlicher pharmazeutische Hilfsstoffe geschieht.

3. Verwendung nach Anspruch 1 oder 2, gekennzeichnet durch gleichzeitige Verwendung von Calciumacetat, Calciumalginat, Calciumcarbonat, Calciumcitrat, Calciumfumarat, Calciumgluconat, Calciumglutamat, Calciumhydroxid, Calciumlactat, Calciummalate, Calciumphytat, Calciumsilicate, Calciumsuccinat, Calciumtartat, die Calciumsalze von Aminosäuren, die Calciumsalze der Ketoanaloga von Aminosäuren oder die Calciumsalze von organischen Säuren, die im pflanzlichen, tierischen und menschlichen Stoffwechsel angetroffen werden und gleichzeitig Magnesiumacetat, Magnesiumalginat, Magnesiumcarbonat, Magnesiumcitrat, Magnesiumfumarat, Magnesiumgluconat, Magnesiumglutamat, Magnesiumhydroxid, Magnesiumlactat, Magnesiummalate, Magnesiumphytat, Magnesiumsilicate, Magnesiumsuccinat, Magnesiumtartat, die Magnesiumsalze von Aminosäuren, die Magnesiumsalze der Ketoanaloga von Aminosäuren oder die Magnesiumsalze von organischen Säuren, die im pflanzlichen, tierischen und menschlichen Stoffwechsel angetroffen werden.

4. Verwendung nach den Ansprüchen 1 bis 3, gekennzeichnet durch Calcium-Magnesiumdoppelsalze, insbesondere durch Calcium-Magnesiumphytat.